# EUROPEAN PATENT APPLICATION

(11) **EP 3 998 020 A1**
(43) Date of publication of application: **18.05.2022**
(21) Application number: 20897554.0
(22) Date of filing: 20.08.2020
(51) Int. Cl.: A61B 5/11, A61B 5/113

(54) **HEART FAILURE DIAGNOSIS DEVICE**

(30) Priority: 02.12.2019 JP 2019218304
(71) Applicant: Sumitomo Riko Company Limited, Komaki-shi, Aichi 485-8550 (JP); OSAKA UNIVERSITY, Suita-shi Osaka 565-0871 (JP)
(72) Inventor: TAKAHASHI, Wataru, Komaki-shi, Aichi 485-8550 (JP); YAMAMOTO, Masanori, Komaki-shi, Aichi 485-8550 (JP); SHIMIZU, Atsuki, Komaki-shi, Aichi 485-8550 (JP); KONDO, Mitsuyoshi, Komaki-shi, Aichi 485-8550 (JP); TAGUCHI, Jun, Komaki-shi, Aichi 485-8550 (JP); ASANOI, Hidetsugu, Suita-shi, Osaka 565-0871 (JP); SAWA, Yoshiki, Suita-shi, Osaka 565-0871 (JP); MIYAGAWA, Shigeru, Suita-shi, Osaka 565-0871 (JP)
(74) Representative: Slingsby Partners LLP
(86) International application number: PCT/JP2020/031438
(87) International publication number: WO 2021/111680

(57) **Abstract**

Provided is a heart failure diagnostic device 10 which uses an indicator generated from a standard deviation of a respiratory frequency of a user P to diagnose heart failure. The heart failure diagnostic device 10 comprises: a flexible piezoelectric sensor sheet 12 outputting a detection signal corresponding to an input vibration; a respiratory signal acquisitor 40 extracting, as a respiratory signal, a signal of a vibration frequency caused by respiration from the detection signal detected by the piezoelectric sensor sheet 12; a power spectrum calculator 44 calculating a power spectrum of a respiratory frequency band from the respiratory signal; and a signal corrector 42 correcting the detection signal such that in the power spectrum, a maximum value of a first-order frequency component of a respiratory waveform is at least 1.5 times a maximum value of a second-order frequency component of the respiratory waveform, thereby obtaining the respiratory signal.

## Description

### TECHNICAL FIELD

The present invention relates to a heart failure diagnostic device that diagnoses heart failure by using an indicator obtained based on a detection signal of respiration.

### BACKGROUND ART

In patients with heart failure, worsening of the medical condition may lead to serious consequences such as death. Thus, grasp of the medical condition, early detection, and the like are important. Therefore, in patients with heart failure, it is necessary to appropriately predict the prognosis of heart failure, to detect the worsening of heart failure at an early stage, to evaluate the severity of heart failure, and the like.

However, although it is conceivable to evaluate the state of heart failure by measuring the sympathetic nerve activity, it is not easy to measure the sympathetic nerve activity. Therefore, for example, Japanese Patent No. JP-B-5679971 (Patent Document 1) proposes a method capable of easily grasping the medical condition of heart failure based on the detection signal of respiration.

The method for diagnosing heart failure disclosed in Patent Document 1 performs frequency analysis from the respiratory waveform of a sleeping subject detected by a respiratory sensor to generate a frequency spectrum. Further, the method extracts a band including the respiratory frequency and generates the respiratory frequency index (RSI), which is the inverse number of the standard deviation of the respiratory frequency, continuously through the night, to obtain the indicator, whereby the symptom of heart failure is grasped by the time transition of the said indicator.

Further, as the respiratory sensor, a respiratory airflow sensor including a thermal sensor attached to the skin surface near the nasal cavity of the subject is exemplified. Meanwhile, in order to further reduce the discomfort of the subject, for example, a pressure-sensing sensor is also proposed to be adopted. The pressure-sensing sensor is placed on the floor surface such as a bed on which the subject sleeps to detect the change in body pressure due to respiration, and does not need to be attached to the body of the subject.

### BACKGROUND ART DOCUMENT

### PATENT DOCUMENT

Patent Document 1: JP-B-5679971

### SUMMARY OF THE INVENTION

### PROBLEM THE INVENTION ATTEMPTS TO SOLVE

However, as examined by the present inventors, it is sufficiently possible to measure respiration based on the change in body pressure detected by the pressure-sensing sensor, while in the case of grasping the symptom of heart failure by obtaining, for example, the above-mentioned respiratory frequency index (RSI) or the like from the frequency spectrum which has been obtained from the respiratory signal detected by the pressure-sensing sensor, it was found that there is room for improvement in accuracy as compared with the case where the respiratory airflow sensor is used.

It is therefore one object of this invention to provide a heart failure diagnostic device of novel structure which is able to improve the accuracy in diagnosing heart failure by the respiratory frequency index (RSI) or the like by using the detection signal of respiration based on the change in body pressure obtained by the piezoelectric sensor. The diagnosis of heart failure in the present invention is not limited to the judgment by a doctor in a narrow sense, but includes, for example, prediction of the prognosis of heart failure, early detection of worsening of heart failure, output of result signals such as evaluation of severity of heart failure, as a determination of symptoms related to heart failure of a subject, and also includes provision of judgment information on symptoms related to heart failure and the like.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a heart failure diagnostic device configured to diagnose heart failure by using an indicator utilizing a standard deviation of a respiratory frequency obtained based on a detection signal of respiration of a lying subject, the heart failure diagnostic device comprising: a piezoelectric sensor sheet having flexibility and configured to output the detection signal corresponding to an input vibration; a respiratory signal acquisitor configured to extract, as a respiratory signal, a signal of a vibration frequency caused by the respiration from the detection signal detected by the piezoelectric sensor sheet; a power spectrum calculator configured to obtain a power spectrum of a respiratory frequency band from the respiratory signal; and a signal corrector configured to correct the detection signal such that in the power spectrum, a maximum value of a first-order frequency component of a respiratory waveform is not smaller than 1.5 times a maximum value of a second-order frequency component of the respiratory waveform to obtain the respiratory signal.

When the present inventor examined the problem of heart failure diagnosis accuracy as described above in a case where a piezoelectric sensor was adopted instead of the respiratory airflow sensor, it was found that the output characteristics peculiar to the piezoelectric sensor were one of the main causes. That is, when diagnosing heart failure based on a detection signal of respiration (a respiratory signal) detected by the piezoelectric sensor, the power spectrum obtained from the respiratory signal is utilized, and the signal in the peak region of the power spectrum existing in the respiratory frequency range will be used as the detection signal of the respiration. Therefore, if the frequency characteristics of the sensitivity of the piezoelectric sensor are not flat, the power spectrum of the respiratory signal will be distorted. This distortion adversely affects the standard deviation of the respiratory signal, resulting in an error in the RSI value. In particular, a general piezoelectric sensor has high-pass filter characteristics generated by insulation resistance and electrostatic capacity of the sensor, and it is difficult to obtain flat frequency characteristics in a low frequency region including the respiratory signal band.

On the other hand, the spectrum of the respiratory signal has a complicated shape including the first-order and high-order frequency components in the low frequency region. Therefore, in the case where the frequency characteristics of the sensitivity of the piezoelectric sensor are the high-pass filter characteristics, the spectrum of the respiratory signal is distorted by the higher-order component being improperly emphasized. Accordingly, it was found that, when calculating the above-mentioned RSI value, there is a possibility of being treated as a respiratory signal including the second-order component, and the respiratory frequency may not be detected accurately. That is, in order to calculate the RSI value with high accuracy, it is necessary to flatten the frequency characteristics of the pressure-sensing sensor.

Here, in the heart failure diagnostic device of the present preferred embodiment, newly adopted is the signal corrector that corrects the first-order frequency component of the respiratory waveform so as to be sufficiently larger than the second-order frequency component in the power spectrum. With this signal corrector, it is possible to acquire the power spectrum by more accurately reflecting the first-order component of the respiratory frequency in the detection signal, which is the original respiratory signal, and in particular, suppressing the adverse effect of the second-order component of the respiratory frequency. Therefore, for example, when diagnosing heart failure by acquiring an indicator related to "regularity of the respiratory cycle" utilizing the standard deviation from the obtained power spectrum and evaluating the time-dependent fluctuation or stability of the respiratory cycle, owing to non-invasive property and non-restrictive property with respect to the subject by adopting the piezoelectric pressure-sensing sensor, the diagnosis accuracy can be improved without loss of good usability eliminating sense of discomfort.

In addition, the piezoelectric type sensor generally has higher sensitivity than other types of sensors such as the capacitance type and the pneumatic type, and is easier to detect respiratory information. Moreover, since there is no bias signal due to static load, the detection signal (the respiratory signal) is not affected by the weight of the subject or how the piezoelectric sensor is laid, and compared with the aforementioned sensors of other types, the piezoelectric type sensor has the advantage of easy signal processing.

Furthermore, the pressure-sensing sensor does not need to restrain the patient. In particular, the piezoelectric pressure-sensing sensor proposed in the present application has high sensitivity and can measure under a bed sheet or under a mattress, thereby reducing a burden on the patient.

A second preferred embodiment provides the heart failure diagnostic device according to the first preferred embodiment, wherein the piezoelectric sensor sheet includes a piezoelectric layer and an electrode layer, and the piezoelectric layer is formed of a rubber elastic body.

In general, the piezoelectric sensor made of ceramics or synthetic resin has a cutoff frequency which is likely to be a lower frequency than that of the piezoelectric sensor made of a rubber elastic body, but the flexibility is low and the patient is likely to feel a sense of discomfort. Therefore, in the heart failure diagnostic device of the present preferred embodiment, while adopting a rubber elastic body whose cutoff frequency tends to be a high frequency as the material of the piezoelectric sensor, by adopting the above-mentioned signal corrector, it is possible to detect the respiratory cycle of the subject with high accuracy. This makes it possible to adopt a flexible piezoelectric sheet while reliably obtaining the detection accuracy of the respiratory signal of the subject and hence the diagnosis accuracy of heart failure, thereby further reducing the sense of discomfort of the subject during use.

A third preferred embodiment provides the heart failure diagnostic device according to the second preferred embodiment, wherein the piezoelectric layer of the piezoelectric sensor sheet is constituted by a high-resistance rubber material having a volume resistivity of not smaller than 10⁹ Ω • cm.

In the heart failure diagnostic device of the present preferred embodiment, a rubber material having a relatively high resistance value is adopted as the piezoelectric layer. This makes it possible for the cutoff frequency on the low frequency side in the piezoelectric sensor, which is generally determined by 1 / (2πRC), to be a lower frequency. Therefore, it is possible to suppress the attenuation of the respiratory signal in the first-order frequency component of the respiratory waveform, and it is also possible to effectively avoid concerns such as noise amplification due to a large correction by the signal corrector.

A fourth preferred embodiment provides the heart failure diagnostic device according to any one of the first to third preferred embodiments, wherein the signal corrector includes a digital filter configured to set a ratio of a maximum power to a minimum power within a range of 0.1 Hz to 0.5 Hz to be not greater than 5.

According to the heart failure diagnostic device of the present preferred embodiment, it is possible to achieve the detection accuracy of the effective detection signal when diagnosing heart failure by, for example, acquiring an indicator related to "regularity of the respiratory cycle" utilizing the standard deviation from the obtained power spectrum and evaluating the time-dependent fluctuation or stability of the respiratory cycle in the respiratory frequency range considered to be effective in diagnosing heart failure.

### EFFECT OF THE INVENTION

With the heart failure diagnostic device according to the present invention, by adopting the piezoelectric sensor sheet, it is possible to accurately diagnose information on heart failure by using an indicator utilizing the standard deviation of the respiratory frequency obtained based on the detection signal detected by the piezoelectric sensor sheet while suppressing the sense of discomfort of the subject at the time of measurement and realizing a good usability.

### BRIEF DESCRIPTION OF THE DRAWINGS

FIG. 1 is a view suitable for explaining a heart failure diagnostic device according to a first practical embodiment of the present invention.
FIG. 2 is a view suitable for explaining a piezoelectric sensor sheet constituting the heart failure diagnostic device shown in FIG. 1.
FIG. 3 is a plan view of a sensor main body constituting the piezoelectric sensor sheet shown in FIG. 2.
FIG. 4 is a cross-sectional view taken along line 4-4 of FIG. 3.
FIG. 5 is a graph showing a specific example of relationship between a digital amount obtained by vibration being input to the piezoelectric sensor sheet and time.
FIG. 6 is a view suitable for explaining a procedure for diagnosing heart failure by using the heart failure diagnostic device shown in FIG. 1.
FIG. 7 is a graph showing a specific example of characteristics of a digital filter constituting a signal corrector.
FIG. 8 is a graph showing a specific example of output characteristics of a sensor after correction in the signal corrector.
FIG. 9 is a graph showing, as a reference, a specific example of output characteristics of the sensor when the digital filter is not adopted in the signal corrector.
FIG. 10 is a graph showing a power spectrum calculated by a power spectrum calculator with respect to a signal corrected in the signal corrector.
FIGS. 11A and 11B are graphs showing specific examples of means for detecting a state in which a user is in bed.

### EMBODIMENTS FOR CARRYING OUT THE INVENTION

Hereinafter, in order to clarify the present invention in more detail, a practical embodiment of the present invention will be described in detail with reference to the drawings.

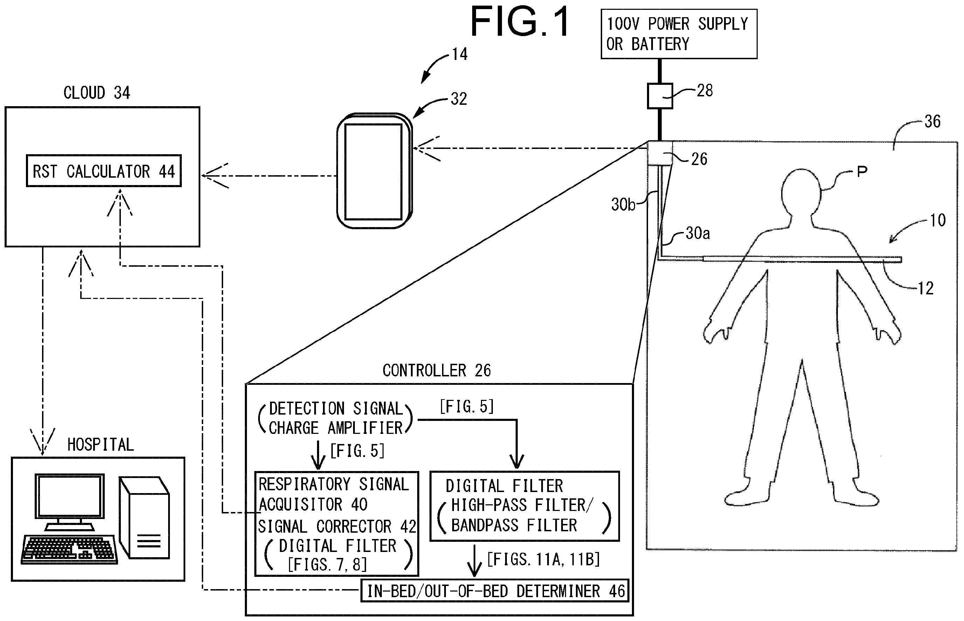

First, FIG. 1 depicts a heart failure diagnostic device 10 according to a first practical embodiment of the present invention. The heart failure diagnostic device 10 includes a piezoelectric sensor sheet 12 to which a minute body movement (a vibration) of the body is input due to respiration, heartbeat and the like of a patient P as a user who is the subject, and which outputs a detection signal corresponding to the input vibration, and an analysis device 14 for analyzing the detection signal output from the piezoelectric sensor sheet 12.

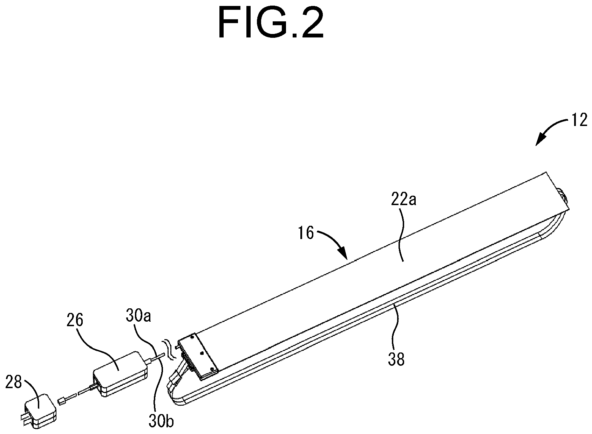

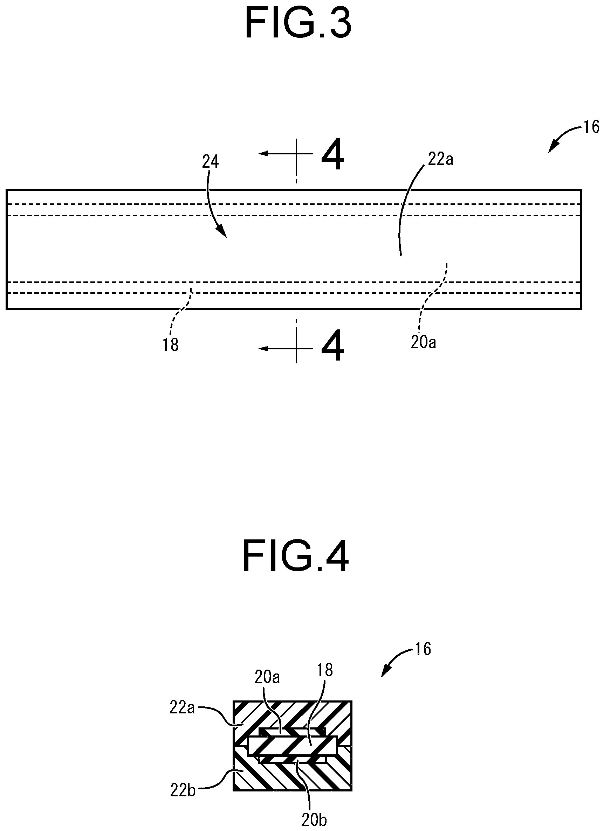

Described more specifically, the piezoelectric sensor sheet 12 has a structure as shown in FIG. 2, for example, and includes a flexible sensor main body 16 having an approximately rectangular sheet shape. As shown in FIGS. 3 and 4, the sensor main body 16 includes a piezoelectric layer 18, a pair of electrode layers 20a, 20b arranged so as to be overlapped on opposite side surfaces of the piezoelectric layer 18 in the direction of sensing pressure, and a pair of protective layers 22a, 22b.

It is possible to adopt, as the material of the piezoelectric layer 18, ceramic, synthetic resin, a rubber elastic body (including elastomer) or the like, but in the present practical embodiment, a rubber elastic body having a volume resistivity pv relatively smaller than that of ceramic and synthetic resin constitutes the piezoelectric layer 18. If the volume resistance is small, the cutoff frequency will be the frequency on the low frequency side in the vibration frequency band caused by respiration on the higher frequency side than the lower limit frequency of the vibration frequency caused by respiration, for example. This may pose a risk that the detection of the first-order frequency component of the respiratory signal may be hindered. For this reason, it is desirable to increase the resistance value of the rubber elastic body so that the cutoff frequency is on the lower frequency side than the lower limit frequency of the vibration frequency caused by respiration, for example. Specifically, the volume resistivity pv of the piezoelectric layer 18 is preferably not smaller than 10⁹ Ω • cm, and more preferably not smaller than 10¹⁰ Ω • cm.

The rubber elastic body adopted as the piezoelectric layer 18 is not limited, but it is preferable to use one or more selected from, for example, crosslinked rubber and thermoplastic elastomer, and examples thereof include, for example, urethane rubber, silicone rubber, nitrile rubber (NBR), hydrogenated nitrile rubber (H-NBR), acrylic rubber, natural rubber, isoprene rubber, ethylene-propylene-diene rubber (EPDM), ethylene-vinyl acetate copolymer, ethylene-vinyl acetate-acrylic acid ester copolymer, butyl rubber, styrene-butadiene rubber, fluororubber, epichlorohydrin rubber, and the like. Further, an elastomer modified by introducing a functional group or the like may be used. As the modified elastomer, for example, a hydrogenated nitrile rubber having one or more selected from a carboxyl group, a hydroxyl group and an amino group is preferable.

Besides, the piezoelectric layer 18 contains piezoelectric particles. Piezoelectric particles are particles of a compound having piezoelectricity. As a piezoelectric compound, a ferroelectric having a perovskite-type crystal structure is known, and it is possible to suitably adopt one kind or a mixture of two or more kinds selected from, for example, barium titanate, strontium titanate, potassium niobate, sodium niobate, lithium niobate, potassium sodium niobate, potassium sodium lithium niobate, lead zirconate titanate (PZT), barium strontium titanate (BST), bismuth lanthanum titanate (BLT), and strontium bismuth tantalate (SBT).

It is preferable that the electrode layers 20a, 20b have flexibility that enables the electrode layers 20a, 20b to deform following the piezoelectric layer 18. Such electrode layers 20a, 20b can be formed of, for example, a conductive material in which a conductive material is mixed with a binder, a conductive fiber, or the like. As the binder, the similar materials to the above-mentioned crosslinked rubber and thermoplastic elastomer constituting the piezoelectric layer 18 can be adopted.

The conductive material mixed in the electrode layers 20a, 20b is not limited, but can be appropriately selected from, for example, metal particles comprising silver, gold, copper, nickel, rhodium, palladium, chromium, titanium, platinum, iron, and alloys thereof or the like, metal oxide particles comprising zinc oxide, titanium oxide or the like, metal carbide particles comprising titanium carbonate, metal nanowires comprising silver, gold, copper, platinum, nickel or the like, and conductive carbon materials such as carbon black, carbon nanotubes, graphite, thin layer graphite, and graphene.

The material of the protective layers 22a, 22b is not limited, but it is desirable that the protective layers 22a, 22b have electric insulation properties, durability, and biocompatibility in addition to flexibility.

In the present practical embodiment, the piezoelectric layer 18, the electrode layers 20a, 20b, and the protective layers 22a, 22b all have a thin rectangular plate shape. The electrode layers 20a, 20b are fixed to opposite sides of the piezoelectric layer 18 in the thickness direction, while the protective layers 22a, 22b are fixed to opposite sides of the piezoelectric layer 18 and the electrode layers 20a, 20b in the thickness direction. With this configuration, the piezoelectric layer 18 and the electrode layers 20a, 20b are embedded inside the protective layers 22a, 22b without being exposed to the outside. With such a structure, the sensor main body 16 is formed so as to have a thin, approximately rectangular sheet shape.

In the central portion of the sensor main body 16 in the width direction, the region where the piezoelectric layer 18 and the electrode layers 20a, 20b overlap in the thickness direction constitutes a pressure sensing part 24. By a load being applied to the pressure sensing part 24, an electric charge will be generated. The pressure sensing part 24 may have a single structure as a whole, or may have a cell structure divided into a plurality of pressure sensing parts in the planar direction.

Further, the piezoelectric sensor sheet 12 of the present practical embodiment includes a controller 26 and a connector 28. The electrode layers 20a, 20b and the controller 26 are electrically connected by wirings 30a, 30b, while the controller 26 is electrically connected to a household power supply, a battery, or the like via the connector 28 and is supplied with power.

The controller 26 includes a charge amplifier that converts the electric charge generated from the piezoelectric sensor sheet 12 into a voltage, and an A/D conversion part that converts the voltage output converted by the charge amplifier into a digital signal. Besides, the controller 26 includes a signal amplification part, and for example, the electric charge generated from the piezoelectric sensor sheet 12, the voltage converted by the charge amplifier, the digital signal converted by the A/D conversion part, and the like can be suitably amplified. Moreover, the controller 26 has a function of transferring the measured data to a smartphone 32 described later by wireless communication such as wi-fi (registered trademark) and bluetooth (registered trademark).

The analysis device 14 of the present practical embodiment includes the smartphone 32. The smartphone 32 is a mobile phone and has a hardware configuration such as a central processing unit (CPU), a RAM, a ROM, and a display serving as a display monitor, and also has a call function, a data communication function and the like that a general smartphone has.

Then, the data transferred from the controller 26 to the smartphone 32 is further transferred from the smartphone 32 to a cloud 34 (on the server). The data sent to the cloud 34 is analyzed by a power spectrum calculator (an RST calculator 44) described later, and an RSI is calculated. The calculated RSI is transferred to the smartphone 32 for confirmation by the patient P himself/herself, or transferred to the hospital for the doctor to judge the progression of heart failure.

Here, in the present practical embodiment, the piezoelectric sensor sheet 12 is arranged so as to extend in the width direction of a bed 36 on which the patient P lies. In the present practical embodiment in particular, the piezoelectric sensor sheet 12 includes a belt part 38 whose length can be adjusted. The belt part 38 is wound around a mattress of the bed 36 so that the piezoelectric sensor sheet 12 is fixed to the top of the mattress, while a bed sheet is placed over the mattress to which the piezoelectric sensor sheet 12 is fixed. By so doing, the patient P lies on the piezoelectric sensor sheet 12 without directly touching the piezoelectric sensor sheet 12, and in the present practical embodiment, the piezoelectric sensor sheet 12 is located near the chest of the patient P.

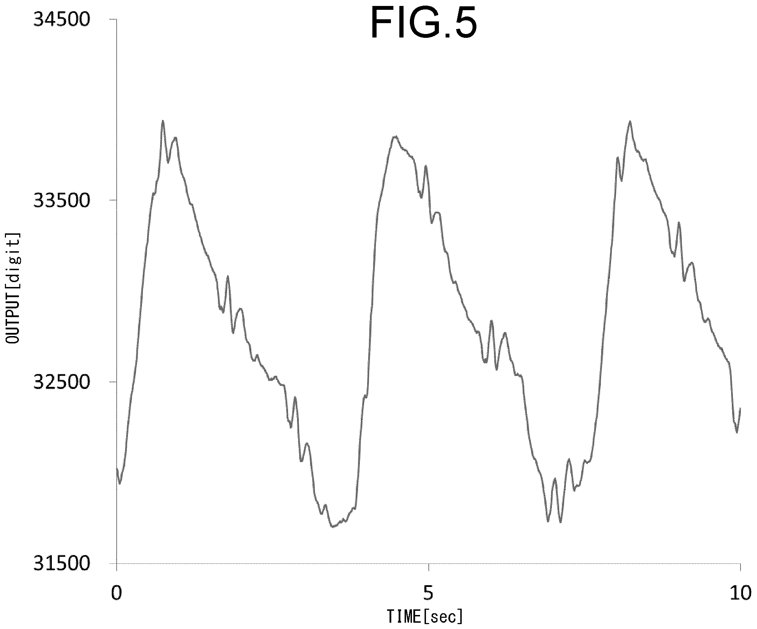

When a minute body movement (a vibration) is input to the pressure sensing part 24 of the piezoelectric sensor sheet 12 due to respiration, heartbeat and the like of the patient P, an electric charge is generated in the piezoelectric layer 18, and the generated electric charge is converted into a voltage by the charge amplifier of the controller 26. Subsequently, the voltage is converted to digital by the A/D converter. A specific example of the relationship between the digital amount detected in the controller 26 and the time is shown in the graph in FIG. 5.

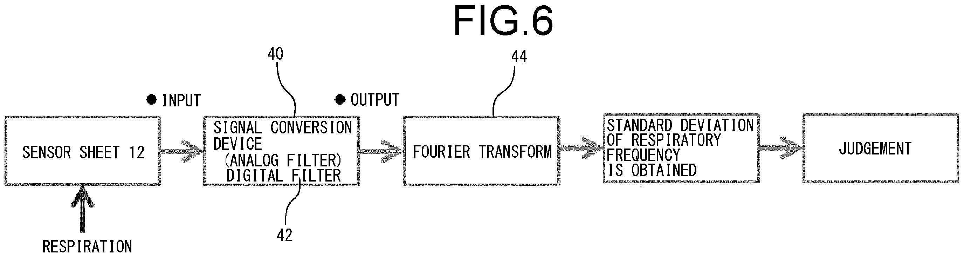

Hereinafter, a specific example of a procedure for analyzing a signal (a detection signal) detected due to respiration and heartbeat of the patient P by the analysis device 14 will be described with reference to FIG. 6.

First, with the patient P lying on the bed 36, a minute body movement (a vibration) due to respiration, heartbeat and the like of the patient P is input to the pressure sensing part 24 of the piezoelectric sensor sheet 12, so that an electric charge (a signal) corresponding to the magnitude (the amplitude) of the vibration is detected by the controller 26 in a time-dependent manner. Then, the signal detected by the controller 26 is output as a respiratory signal via a digital filter.

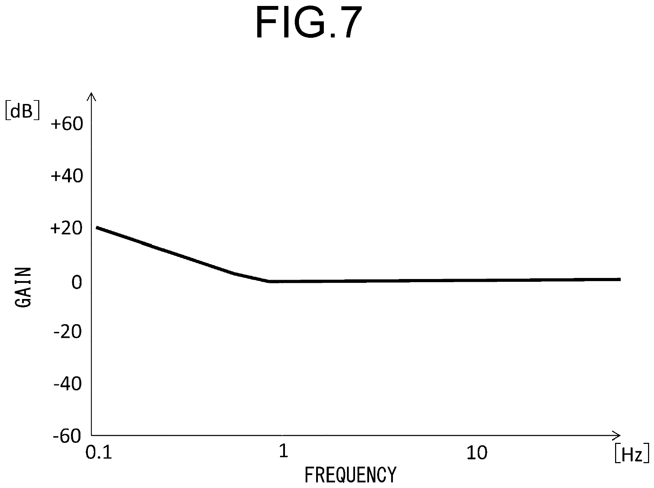

That is, in order to make the output characteristics of the piezoelectric sensor sheet 12 approximately flat by the digital filter, a correction process is performed on the data converted into the digital signal (the detection signal) so as to amplify the low frequency side or to attenuate the high frequency side. The correction process by the digital filter may be performed by the controller 26, or may be performed by the smartphone 32 or the cloud 34 in which an appropriate program (an application) is installed. Although the numerical formula (the correction formula) or the like indicating the specific process of the digital filter is not limited, FIG. 7 shows a specific example of the output characteristics of the digital filter.

Since the piezoelectric sensor sheet 12 has some variations in production, it is preferable to set the correction formula in consideration of such variations. Therefore, the correction formula of the digital filter may be appropriately set at the time of the first measurement, or every time of measurement, or with a predetermined measurement interval, a time interval, or the like, depending on the obtained detection signal or the like.

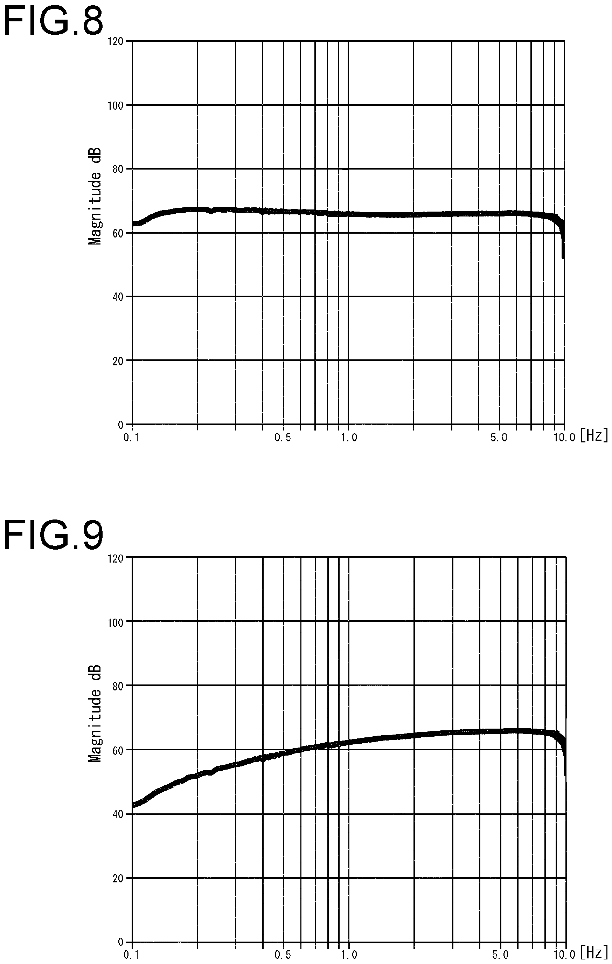

As a result, the signal detected by the controller 26 is extracted as a corrected detection signal (a respiratory signal) with the output characteristics shown in FIG. 8 via the digital filter.

As a reference, FIG. 9 shows the output characteristics of the piezoelectric sensor sheet 12 when the output is performed bypassing the digital filter. As shown in FIG. 9, by adopting a rubber elastic body as the piezoelectric layer 18, it can be seen that there is a cutoff frequency near 0.5 Hz on the low frequency side, and the output near 0.1 Hz, for example, is greatly reduced. On the other hand, by amplifying the output on the lower frequency side than the cutoff frequency in the piezoelectric sensor sheet 12 with the digital filter, as shown in FIG. 8, for example, the output characteristics within a range of 0.1 Hz to 0.5 Hz approximately corresponding to the vibration frequency caused by human respiration are set to be approximately flat, and the ratio of the maximum power to the minimum power within a range of 0.1 Hz to 0.5 Hz is not greater than 5 (not greater than 5 dB).

Incidentally, if the ratio of the maximum power to the minimum power within a range of 0.1 Hz to 0.5 Hz is greater than 5, there is a risk that, for example, the harmonic of the respiratory frequency (the second-order frequency component) may have a large influence when calculating an RST, which will be described later, and the RST will not be calculated accurately.

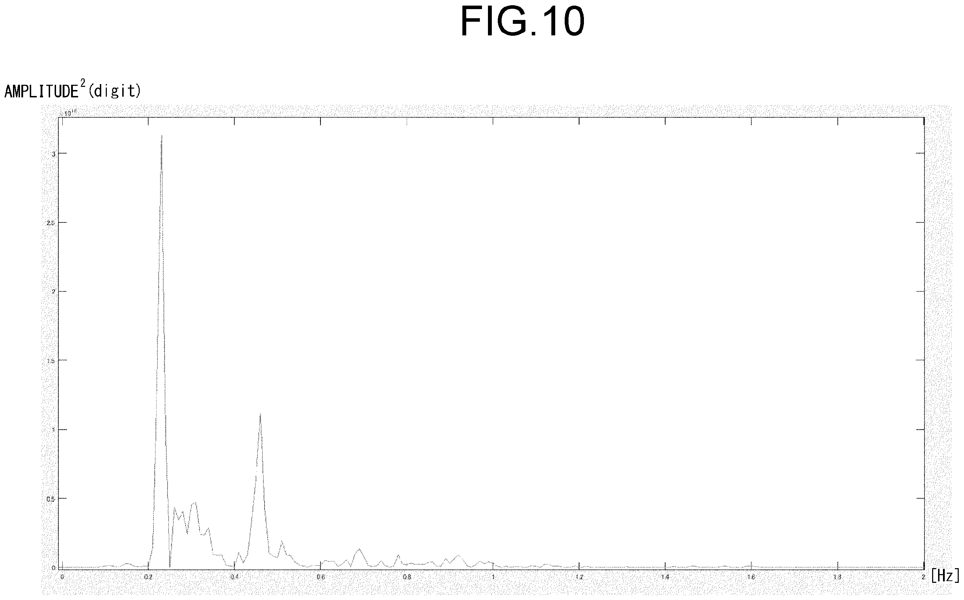

From the respiratory signal corrected so that the low frequency side is amplified in this way and output, the power spectrum is calculated by frequency analysis at least in the frequency including the respiratory frequency band (for example, 0.1 Hz to 0.5 Hz). The result is shown in FIG. 10.

In the present practical embodiment, in the power spectrum shown in FIG. 10, the peak (the mountain part) of the first-order frequency component based on the fundamental wave of the respiratory waveform appears at a position of about 0.25 Hz, and the peak (the mountain part) of the second-order frequency component, which is the harmonic, appears at a position of about 0.5 Hz.

In such a power spectrum, the maximum value of the first-order frequency component is not smaller than 1.5 times the maximum value of the second-order frequency component. If the maximum value of the first-order frequency component is smaller than 1.5 times the maximum value of the second-order frequency component, there is a risk that the second-order frequency component may have a large influence and the RST described later will not be calculated accurately.

Then, with respect to the obtained power spectrum, as described in above-mentioned Patent Document 1, the standard deviation (SD) of the respiratory frequency of the patient P is calculated. Then, by acquiring an inverse number (RSI) of this standard deviation (SD), the RST (the respiratory stability time) serving as an indicator indicating the stability of the respiratory cycle and hence the severity of the heart failure disease can be obtained. The severity and stage of the heart failure disease can be judged by determining, for example, whether the RST value is larger or smaller than a predetermined value, or larger or smaller than the RST value at the time of the previous measurement, or the like.

That is, in the present practical embodiment, the detection signal converted by the charge amplifier is converted into a respiratory signal through the digital filter adjusted so that the frequency characteristics are approximately flat, and the respiratory signal is transferred to the cloud 34 through the Internet communication via the smartphone 32. Then, the transferred data is converted into a power spectrum, and the standard deviation of the respiratory waveform is calculated to obtain the RST. Therefore, in the present practical embodiment, a respiratory signal acquisitor 40 that extracts the signal of the vibration frequency caused by respiration from the detection signal as the respiratory signal, and a signal corrector 42 that corrects the detection signal detected by the controller 26 (the piezoelectric sensor sheet 12) with the digital filter to obtain a respiratory signal are constituted by at least one of the controller 26, the smartphone 32, and the cloud 34 including an appropriate program (an application) (in FIG. 1, the respiratory signal acquisitor 40 and the signal corrector 42 are provided in the controller 26). Further, the RST calculator 44 serving as a power spectrum calculator that calculates the power spectrum from the respiratory signal comprises a program on the cloud 34.

Meanwhile, the heart failure disease has been found to correlate with sleep quality. Therefore, in addition to the aforementioned RST information, it is preferable to obtain information on whether the patient P is in the bed 36 or out of the bed 36. In the determination of whether the person is in bed or out of bed is performed by, for example, converting the detection signal converted by the charge amplifier through a digital filter (for example, a high-pass filter for extracting a frequency component of 4 Hz or higher, or a bandpass filter for extracting a frequency component of 0.8 Hz to 2 Hz described later), and determining whether the signal means the patient is in bed or out of bed through an in-bed/out-of-bed determiner 46. The determination signal is transferred to the cloud 34 via the Internet communication. That is, in the present practical embodiment, the in-bed/out-of-bed determiner 46 is constituted by at least one of the controller 26, the smartphone 32, and the cloud 34 including an appropriate program (an application) (in FIG. 1, the in-bed/out-of-bed determiner 46 is provided in the controller 26).

Then, the RST and the in-bed or out-of-bed information obtained in the above-described way may be transferred to a system that can be seen by a doctor at a hospital, or may be transferred to the smartphone 32 of the patient P, via the Internet communication. This allows the doctor to determine if the patient P has a tendency toward heart failure by looking at the RST and the in-bed or out-of-bed information, and if the patient is determined to have a tendency toward heart failure, appropriate treatments such as drug intervention, outpatient treatment, or the like are given.

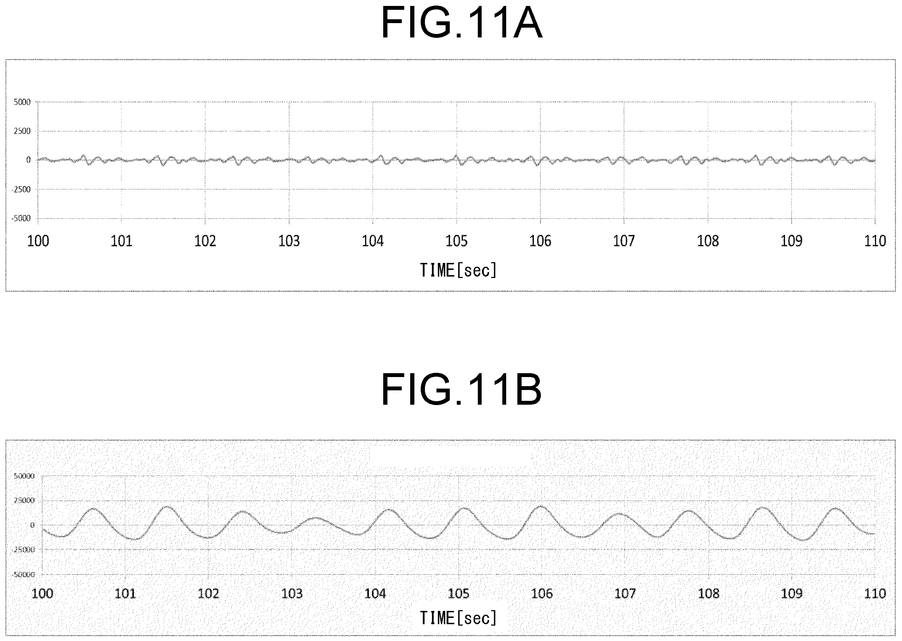

The specific means for detecting whether the patient P is in bed or out of bed with respect to the bed 36 is not limited, but for example, the detection whether the patient P is in bed or out of bed with respect to the bed 36 may be performed based on the detection signal detected by the piezoelectric sensor sheet 12 as described above. That is, the heartbeat of a person is about 1 Hz, but the vibration of the body due to the heartbeat (the ballistocardiographic motion) is about 4 Hz or greater. By the ballistocardiographic motion being detected as shown in FIG. 11A via a high-pass filter that extracts a frequency component of 4 Hz or greater with respect to the detected signal, the patient P may be determined to be in the bed 36. Alternatively, by the frequency component of 4 Hz or greater caused by the ballistocardiographic motion passing through a bandpass filter that further extracts a frequency component of 0.8 Hz to 2 Hz, it is also possible to detect the heartbeat as shown in FIG. 11B, and the detection of the heartbeat may determine that the patient P is in the bed 36. If, for example, these ballistocardiographic motion or heartbeat are not detected, the patient P is determined to be out of bed.

The severity of the heart failure disease may be judged by determining whether the patient P is in bed or out of bed with respect to the bed 36 as described above, in addition to the RST information. That is, as described in International Publication No. WO 2011/019091, it has been found that patients with heart failure have poor sleep quality. Accordingly, for example, if the patient is in the bed 36 and lying thereon during the daytime (regardless of whether or not he/she is asleep) even though he/she has sufficient sleep time at night, the information of in-bed time, out-of-bed time and the like can be of some help to judge the severity of the heart failure disease. In particular, since there is a difference in RST during sleep between a heart failure patient and a healthy person, the severity of the heart failure disease may be judged by, for example, comparing the RST of the patient P who is presumed to fall asleep with the RST of a healthy person after a predetermined time such as 30 minutes and 1 hour after the detection of being in bed.

Such a determination whether the patient P is in bed or out of bed with respect to the bed 36 may be performed based on the detection signal detected by the piezoelectric sensor sheet 12 as described above. However, the patient P may alternatively be determined to be in the bed 36 by separately providing a pressure sensor described in, for example, Japanese Unexamined Patent Publication No. JP-A-2015-008920 or International Publication No. WO 2015/186182 to detect the body pressure of the patient P.

The heart failure diagnostic device 10 of the present practical embodiment as described above adopts the flexible piezoelectric sensor sheet 12, in which the piezoelectric layer 18 is made of a rubber elastic body, as a sensor for detecting the vibration caused by respiration of the patient P. With this configuration, the piezoelectric sensor sheet 12 can easily deform according to the body shape of the patient P, thereby reducing the possibility that the patient P lying on the bed 36 feels unnatural sense or discomfort.

However, by forming the piezoelectric layer 18 with a rubber elastic body, it is difficult to obtain a signal on the low frequency side. Therefore, by providing the signal corrector 42 and performing correction such as amplifying the low frequency side in the power spectrum, the influence of harmonics can be avoided, thereby more accurately calculating the RST. This makes it possible to more reliably judge the severity of the heart failure disease.

Specifically, since the piezoelectric layer 18 is made of a rubber elastic body having a high resistance of not smaller than 10⁹ Ω • cm, a signal on the low frequency side can be obtained more stably.

Further, by providing a digital filter in the signal corrector 42 to amplify the low frequency side of the respiratory signal, a signal on the low frequency side, which is difficult to obtain due to the piezoelectric layer 18 being formed of a rubber elastic body, can be stably obtained, thereby performing calculation of the RST and judgement of heart failure more accurately.

Moreover, in the present practical embodiment, the severity of heart failure and the like are determined not only by the RST but also by the detection result of the patient P being in bed and out of bed with respect to the bed 36. In the present practical embodiment in particular, the patient P being in bed and out of bed with respect to the bed 36 is detected based on the detection signal detected by the piezoelectric sensor sheet 12. Thus, it is not necessary to separately provide a special configuration, and more accurate diagnosis can be made with a simple structure.

Furthermore, since there is a possibility that the sensor sensitivity may vary depending on variations in production of the piezoelectric sensor sheet 12 and the outside environment, etc., by setting the correction formula in consideration of such variations, it is also possible to suppress the variations in the sensor sensitivity.

Although the practical embodiment of the present invention has been described above, the present invention is not limitedly interpreted based on the specific description in the practical embodiment, but may be embodied with various changes, modifications and improvements which may occur to those skilled in the art.

Whereas a digital filter is adopted as a means for amplifying and correcting a signal on the low frequency side in the preceding practical embodiment, an analog filter may alternatively be adopted. By so doing, the reactivity (the sensitivity) can be improved. However, as in the preceding practical embodiment, by adopting the digital filter, the number of parts and the cost can be reduced. Meanwhile, in the analog filter, the characteristics change due to temperature and humidity, the characteristics vary depending on the lots of parts, or the like. Thus, the digital filter is preferred.

The user of the heart failure diagnostic device according to the present invention is not limited to, for example, a patient who is hospitalized or going to the hospital for treatment. It would also be acceptable to use the heart failure diagnostic device at home to calculate the RST, so as to grasp the severity of the heart failure disease from the result, and to receive an appropriate treatment at a hospital or the like as needed.

### KEYS TO SYMBOLS

10 heart failure diagnostic device
12 piezoelectric sensor sheet
14 analysis device
16 sensor main body
18 piezoelectric layer
20a, 20b electrode layer
22a, 22b protective layer
24 pressure sensing part
26 controller
28 connector
30a, 30b wiring
32 smartphone
34 cloud
36 bed
38 belt part
40 respiratory signal acquisitor
42 signal corrector
44 RST calculator (power spectrum calculator)
46 in-bed/out-of-bed determiner
P patient (user)

## Claims

1. A heart failure diagnostic device configured to diagnose heart failure by using an indicator utilizing a standard deviation of a respiratory frequency obtained based on a detection signal of respiration of a lying subject, the heart failure diagnostic device comprising:
a piezoelectric sensor sheet having flexibility and configured to output the detection signal corresponding to an input vibration;
a respiratory signal acquisitor configured to extract, as a respiratory signal, a signal of a vibration frequency caused by the respiration from the detection signal detected by the piezoelectric sensor sheet;
a power spectrum calculator configured to obtain a power spectrum of a respiratory frequency band from the respiratory signal; and
a signal corrector configured to correct the detection signal such that in the power spectrum, a maximum value of a first-order frequency component of a respiratory waveform is not smaller than 1.5 times a maximum value of a second-order frequency component of the respiratory waveform to obtain the respiratory signal.

2. The heart failure diagnostic device according to claim 1, wherein the piezoelectric sensor sheet includes a piezoelectric layer and an electrode layer, and the piezoelectric layer is formed of a rubber elastic body.

3. The heart failure diagnostic device according to claim 2, wherein the piezoelectric layer of the piezoelectric sensor sheet is constituted by a high-resistance rubber material having a volume resistivity of not smaller than 10⁹ Ω • cm.

4. The heart failure diagnostic device according to any one of claims 1-3, wherein the signal corrector includes a digital filter configured to set a ratio of a maximum power to a minimum power within a range of 0.1 Hz to 0.5 Hz to be not greater than 5.
